(19) Europäisches Patentamt
European Patent Office
Office européen des brevets

(11) **EP 4 501 307 A1**

(12) **EUROPEAN PATENT APPLICATION**
published in accordance with Art. 153(4) EPC

(43) Date of publication:
**05.02.2025 Bulletin 2025/06**

(21) Application number: **23781105.4**

(22) Date of filing: **31.03.2023**

(51) International Patent Classification (IPC):
**A61K 8/44** (2006.01)  **A61K 8/72** (2006.01)
**A61Q 1/00** (2006.01)  **A61Q 19/00** (2006.01)

(52) Cooperative Patent Classification (CPC):
**A61K 8/44; A61K 8/72; A61Q 1/00; A61Q 19/00**

(86) International application number:
**PCT/JP2023/013708**

(87) International publication number:
**WO 2023/191097 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.03.2022 JP 2022058803**

(71) Applicant: **AJINOMOTO CO., INC.**
**Chuo-ku**
**Tokyo 104-8315 (JP)**

(72) Inventors:
- **NAKAJIMA Satoshi**
  **Kawasaki-shi, Kanagawa 210-0801 (JP)**
- **BISWAS Shuvendu**
  **Tokyo 104-8315 (JP)**
- **YAMAZAKI Tsuyoshi**
  **Kawasaki-shi, Kanagawa 210-8681 (JP)**
- **MIZOGUCHI Kota**
  **Kawasaki-shi, Kanagawa 210-8681 (JP)**

(74) Representative: **Mewburn Ellis LLP**
**Aurora Building**
**Counterslip**
**Bristol BS1 6BX (GB)**

(54) **POWDER COMPOSITION**

(57)     The present invention provides a product which has excellent moldability, while achieving a soft texture equivalent to or better than that of nylon. The present invention relates to a powder composition which contains (A) an acyllysine that has a median diameter of the volume base distribution of 9 μm or less, and (B) a powder of a polymer that uses, as a monomer, at least one substance that is selected from among an amino acid, a hydroxycarboxylic acid, a uronic acid, a polyvalent carboxylic acid and a polyhydric alcohol and/or (C) a polymer that uses, as a monomer, a sugar or glucosamine.

EP 4 501 307 A1

**Description**

Technical Field

The present invention relates to a powder composition, and more particularly, to a powder composition for cosmetics or external preparations for skin.

Background Art

Spherical powders are used in many cosmetics to achieve a soft focus effect (an effect in which reflected light from the powder surface is scattered in multiple directions to blur the surface to which the powder is applied) and a rolling feel. In addition to these effects, spherical powders made of resins such as nylon and polyurethane can also impart softness and moisture, and therefore, are often blended to produce cosmetics with a good feel. However, since these resin-based spherical powders are not biodegradable, they remain in the environment when released into the environment, which is a concern from the viewpoint of bioaccumulation. In order to solve this problem, the use of naturally occurring spherical powders (e.g., cellulose, silica, starch, etc.) has been investigated. However, since these powders have a hard feel and a strong dry feel, spherical powders with a better feel and function have been demanded. Attempts have been made to impart a moist and silky feel to naturally occurring powders by mixing lauroyl lysine with the naturally occurring spherical powders. For example, U.S. Patent No. 5,662,937 describes a method in which the feel of cornstarch is improved by dissolving lauroyl lysine in a basic solvent and mixing it with cornstarch. However, when a basic solution of lauroyl lysine is mixed with a naturally occurring spherical powder, there is a problem that the basic solution causes the naturally occurring spherical powder to swell, dissolve, or deteriorate. Furthermore, there is a problem that complicated steps such as washing and drying of the basic solution are required, making industrial production difficult. On the other hand, Japanese Patent Application No. 2019-030775 states that by mixing and pulverizing cornstarch and lauroyl lysine with a ball mill, it was possible to obtain spherical powders that are excellent in feel and slipperiness during application, can impart a moist feeling, and are free from tugging. Similarly, Japanese Unexamined Patent Application Publication No. 2020-75878 states that by mixing and pulverizing fine cellulose particles adjusted under special conditions with lauroyl lysine in a ball mill, it was possible to obtain spherical powders with high moist feeling, slipperiness, softness, and no tugging feeling. However, there is a problem in that the naturally occurring spherical powder itself is pulverized in the pulverization and mixing in the ball mill.

Citation List

Patent Literature

Patent Literature 1: U.S. Patent No. 5,662,937
Patent Literature 2: Japanese Unexamined Patent Application Publication No. 2019-030775
Patent Literature 3: Japanese Unexamined Patent Application Publication No. 2020-75878

Summary of Invention

Furthermore, these methods have not been able to achieve a soft feel comparable to that of nylon. Also, it was far from the feel of polyurethane, which is said to have a softer feel than nylon. Similar problems have arisen in the attainment of sliminess.
As a result of intensive studies, the present inventors have found that by mixing acyllysine having a median diameter of volume-based distribution of 9 $\mu$m or less with a powder of a polymer having at least one selected from amino acids, hydroxycarboxylic acids, uronic acids, polyvalent carboxylic acids, and polyhydric alcohols as a monomer, it is possible to obtain a powder capable of providing a softer feel and sliminess than nylon. The present inventors have also found that the obtained powder is further excellent in moist feeling, adhesion to the skin, and can also achieve a silky feel, thereby completing the present invention. That is, the present invention is as follows.

[1] A powder composition comprising

(A) acyllysine having a median diameter of volume-based distribution of 9 $\mu$m or less; and
(B) a powder of a polymer having at least one selected from amino acids, hydroxycarboxylic acids, uronic acids, polyvalent carboxylic acids, and polyhydric alcohols as a monomer; and/or (C) a polymer having a sugar or glucosamine as a monomer.

[2] The composition according to [1], wherein the polymer of (B) is at least one selected from proteins, hydroxycarboxylic acid-based polyesters, polyhydroxyalkanoic acids, diol carboxylic acid-based polyesters, and alginic acid.

[3] The composition according to [1] or [2], wherein the (A) acyllysine is lauroyl lysine or octanoyl lysine.

[4] The composition according to any one of [1] to [3], further comprising (C) a polymer having a sugar or glucosamine as a monomer.

[5] The composition according to any one of [1] to [4], wherein the (C) is at least one selected from cellulose, starch, chitin, and chitosan.

[6] The composition according to any one of [1] to [5], wherein the polymer of the (B) powder exhibits biodegradability in any one of ASTM D6400 biodegradability test, ISO 17088 test method, ISO-TG301F test method, and OCED 306 test method.

[7] The composition according to any one of [1] to [6], wherein the (A) acyllysine has a bulk density of 0.38 g/mL or less.

[8] The composition according to any one of [1] to [7], wherein

the content of the (A) is 3 to 50% by mass,
the content of the (B) is 5 to 97% by mass, and
the content of the (C) is 0 to 92% by mass,
based on 100% by mass of the total amount of the powder composition.

[9] The composition according to any one of [1] to [8], further comprising an inorganic powder.

[10] The composition according to [9], wherein

the content of the (A) is 3 to 50% by mass,
the content of the (B) is 0 to 97% by mass,
the content of the (C) is 0 to 92% by mass, and
the content of the inorganic powder is 0 to 85% by mass,
based on 100% by mass of the total amount of the powder composition.

[11] The composition according to [9] or [10], wherein

the content of the (A) is 3 to 50% by mass,
the content of the (B) is 6 to 30% by mass, and
the content of the inorganic powder is 35 to 90% by mass,
based on 100% by mass of the total amount of the powder composition.

[12] A method for producing the composition according to any one of [1] to [11], comprising mixing:

(A) acyllysine having a median diameter of volume-based distribution of 9 $\mu$m or less,
(B) a powder of a polymer having at least one selected from amino acids, hydroxycarboxylic acids, uronic acids, polyvalent carboxylic acids, and polyhydric alcohols as a monomer, and/or (C) a polymer having a sugar or glucosamine as a monomer, and

optionally, an inorganic powder.

[13] A method for producing the composition according to any one of [1] to [11], comprising mixing (A) acyllysine having a median diameter of volume-based distribution of 9 $\mu$m or less, (B) a powder of a polymer having at least one selected from amino acids, hydroxycarboxylic acids, uronic acids, polyvalent carboxylic acids, and polyhydric alcohols as a monomer, and (C) a polymer having a sugar or glucosamine as a monomer.

[14] The production method according to [12] or [13], wherein the mixing comprises mixing by dry mixing.

[15] The production method according to any one of [12] to [14], wherein the mixing comprises mixing with a mixer for 120 minutes or less.

Description of Embodiments

The powder composition of the present invention comprises (A) acyllysine having a median diameter of volume-based distribution of 9 $\mu$m or less, (B) a powder of a polymer having at least one selected from amino acids, hydroxycarboxylic acids, uronic acids, polyvalent carboxylic acids, and polyhydric alcohols as a monomer, and/or (C) a polymer having a sugar or glucosamine as a monomer.

The (A) acyllysine contained in the powder composition of the present invention has a median diameter of volume-based distribution of 9 μm or less; preferably 6 μm or less from the viewpoint of improving adhesion; more preferably 5 μm or less from the viewpoint of further improving adhesion and feel; and even more preferably 4 μm or less from the viewpoint of improving adhesion and shortening the production time.

The median diameter of the (A) acyllysine of the present invention in terms of number-based distribution is preferably 4 μm or less.

The median diameter of the acyllysine can be determined by measuring the particle size distribution on a number basis or a volume basis using a laser diffraction/scattering particle size distribution analyzer. The median diameter means the particle size at the point where the cumulative undersize distribution curve intersects the horizontal axis at 50%. "Number-based distribution" refers to a particle size distribution in which, when calculating the frequency of each particle size of the particle size distribution, the number of particles is counted and calculated based on the number of particles, and "volume-based distribution" refers to a particle size distribution in which, when calculating the frequency of each particle size of the particle size distribution, the volume of particles assumed to be spherical is counted and calculated based on the value.

The bulk density of the acyllysine is preferably 0.38 g/mL or less. The bulk density is preferably 0.01 to 0.38 g/mL, more preferably 0.05 to 0.38 g/mL, and even more preferably 0.1 to 0.36 g/mL.

The bulk density of the acyllysine can be measured by the following measuring method.

The acyllysine is disaggregated by stirring with a mixer for 2 minutes or more, and the bulk density of the obtained crystals is measured using a powder flowability analyzer (for example, Powder Rheometer FT-4 (manufactured by Freeman Technology™)). Specifically, after a certain amount of acyllysine is measured into a holder, conditioning is performed according to the usage procedure, and the bulk density is measured from the volume after conditioning and the mass of the acyllysine by the following formula.

$$\text{Bulk density} = \text{mass after conditioning/volume after conditioning (g/mL)}$$

The (A) acyllysine having a median diameter of volume-based distribution of 9 μm or less can be obtained by a production method in which a basic solution of acyllysine is added dropwise to a solution such as hydrochloric acid to crystallize, or a pulverization method using a machine or the like. The acyllysine having a median diameter of volume-based distribution of 9 μm or less can be obtained, for example, by the method described in WO2020/262367.

The acyl group of the (A) acyllysine is a saturated or unsaturated fatty acid acyl group having 8 to 22 carbon atoms, such as octanoyl, lauroyl, myristoyl, palmitoyl, stearoyl, octyldodecyl, oleyl, behenyl, coconut oil fatty acid acyl, palm kernel oil fatty acid acyl, beef tallow fatty acid acyl, and the like; and is preferably at least one selected from the group consisting of lauroyl and octanoyl from the viewpoint of general availability. That is, the acyllysine is preferably a saturated fatty acid acyl group having 8 to 12 carbon atoms, more preferably lauroyl lysine or octanoyl lysine, and even more preferably lauroyl lysine.

The powder of (B) polymer contained in the powder composition of the present invention has at least one selected from amino acids, hydroxycarboxylic acids, uronic acids, polyvalent carboxylic acids, and polyhydric alcohols as a monomer.

Examples of the amino acid include isoleucine, leucine, lysine, methionine, phenylalanine, threonine, tryptophan, valine, histidine, tyrosine, cysteine, aspartic acid, asparagine, serine, glutamic acid, glutamine, proline, glycine, alanine, arginine, and theanine, and may be composed of a plurality of types.

Examples of the hydroxycarboxylic acid include hydroxybutyric acid, hydroxyvaleric acid, hydroxycaproic acid, hydroxycaprylic acid, R-3-hydroxybutanoic acid (3HB), R-3-hydroxyhexanoic acid (3HH), lactic acid, and glycolic acid.

Examples of the uronic acid include mannuronic acid, glucuronic acid, and galacturonic acid.

Examples of the polyvalent carboxylic acid include succinic acid, adipic acid, and terephthalic acid.

Examples of the polyhydric alcohol include glycerin, pentylene glycol, dipropylene glycol, butylene glycol (1,3-butanediol), propylene glycol (1,2-propanediol), 1,4-butanediol, and 1,3-propanediol.

The average particle size of the powder of (B) polymer is preferably 50 μm or less, more preferably 1 to 35 μm, and even more preferably 2 to 20 μm. Here, the average particle size of the powder of (B) polymer is specified as in the JIS standard (Z8819-2: 2019).

Examples of the powder of (B) polymer include protein, hydroxycarboxylic acid-based polyester, polyhydroxyalkanoic acid, diol carboxylic acid-based polyester, and alginic acid.

Examples of the protein include silk, wool, and feathers. As the protein powder, silk powder is preferable. The silk powder is not particularly limited, and silk powder obtained by a known method can be used. Commercially available silk powder can be used, such as Silk Powder manufactured by Izumi Senko Co., Ltd.; N-Fibroin manufactured by Nagasuna Mayu Co., Ltd.; and Silkgen G Powder manufactured by Ichimaru Pharcos Co., Ltd.

The average particle size of the silk powder is preferably 50 $\mu$m or less, more preferably 1 to 35 $\mu$m, and even more preferably 2 to 20 $\mu$m as the equivalent spherical diameter. Here, the equivalent spherical diameter of the silk powder is specified as in the JIS standard (Z8819-2: 2019).

Examples of the hydroxycarboxylic acid-based polyester include polylactic acid, polyglycolic acid, polycaprolactone, and glycolic acid-caprolactone copolymer.

Examples of the polyhydroxyalkanoic acid include polyhydroxybutyric acid (PHB), polyhydroxyvaleric acid, poly(3-hydroxybutyric acid-co-3-hydroxyvaleric acid) (PHBV), polyhydroxycaproic acid, polyhydroxycaprylic acid, PHBH composed of R-3-hydroxybutanoic acid (3HB) and R-3-hydroxyhexanoic acid (3HH), and modified polyvinyl alcohol.

Examples of the diol carboxylic acid-based polyester include polyethylene succinate, polybutylene succinate, polyethylene terephthalate succinate, and polybutylene adipate terephthalate.

Examples of the alginic acid include calcium alginate.

As the powder of (B) polymer, a powder of a biodegradable polymer that exhibits biodegradability in a biodegradability test is preferable. As the biodegradability test method, ASTM D6400 test method, ISO 17088 test method, ISO-TG301F test method, or OCED 306 test method is used. In the present invention, "exhibiting biodegradability" means exhibiting biodegradability in at least one of these test methods. Examples of the biodegradable polymer include polylactic acid, polyhydroxybutyric acid (PHB), poly(3-hydroxybutyric acid-co-3-hydroxyvaleric acid) (PHBV), polyglycolic acid, polyethylene succinate, polybutylene succinate, polybutylene succinate adipate, polyethylene terephthalate succinate, and polybutylene adipate terephthalate. Commercially available biodegradable polymer powders can be used, such as Ecosoft 608XF, Ecosoft 608, Biosoft 915, and NatureMatte 31 manufactured by Micro Powders, Inc.; and TECHPOLYMER BIO EF-A manufactured by Sekisui Plastics Co., Ltd.

The average particle size of the biodegradable polymer powder is preferably 50 $\mu$m or less, more preferably 1 to 35 $\mu$m, and even more preferably 2 to 20 $\mu$m. Here, the average particle size of the biodegradable polymer powder is specified as in the JIS standard (Z8819-2: 2019).

The powder composition of the present invention may further include (C) a polymer having a sugar or glucosamine as a monomer.

Examples of the sugar include glucose.

Examples of the glucosamine include glucosamine and N-acetylglucosamine.

Examples of the (C) polymer include cellulose, starch, modified starch, cellulose derivative, chitin, and chitosan. As the (C) polymer, cellulose and starch are preferable.

Examples of the cellulose include plate-shaped cellulose, spherical cellulose, and amorphous cellulose, with spherical cellulose being preferred. Here, spherical includes true spherical, substantially spherical, and elliptical. The area circularity of the spherical cellulose is preferably 0.62 or more, and more preferably 0.68 or more. The addition of cellulose can impart softness. Moreover, it is preferable to select spherical cellulose in order to maintain the rolling feel.

Examples of the spherical cellulose include spherically crystallized cellulose and non-crystalline cellulose.

The average particle size of the spherical cellulose is preferably 2 to 30 $\mu$m or 8 to 30 $\mu$m, more preferably 3 to 20 $\mu$m or 8 to 20 $\mu$m, and even more preferably 5 to 12 $\mu$m or 8 to 16 $\mu$m. Here, the average particle size of the spherical cellulose is specified as in the JIS standard (Z8819-2: 2019).

As the cellulose, those registered as crystalline cellulose, cellulose acetate, and lignin under the INCI name can be used. Commercially available cellulose can be used, such as TEGO Feel Green manufactured by Evonik Industries AG; CELLULOBEADS D-10 and CELLULOBEADS D-5 manufactured by Daito Kasei Kogyo Co., Ltd.; and crystalline cellulose manufactured by JRS.

Examples of the starch include starch derived from rice, corn, potato, tapioca, barley, and the like, as well as pregelatinized starch and partially pregelatinized starch thereof. Starch derived from tapioca, barley, and rice is preferable. Starch derived from tapioca or barley is more preferable. The shape of the starch may be spherical or amorphous, with spherical being preferred. Here, spherical includes true spherical, substantially spherical, and elliptical. The median diameter of volume-based distribution of the starch is preferably 5 to 39 $\mu$m, more preferably 5 to 30 $\mu$m, and even more preferably 8 to 20 $\mu$m.

The median diameter of the starch can be determined by measuring the particle size distribution on a number basis or a volume basis using a laser diffraction/scattering particle size distribution analyzer. The median diameter means the particle size at the point where the cumulative undersize distribution curve intersects the horizontal axis at 50%. "Number-based distribution" refers to a particle size distribution in which, when calculating the frequency of each particle size of the particle size distribution, the number of particles is counted and calculated based on the number of particles, and "volume-based distribution" refers to a particle size distribution in which, when calculating the frequency of each particle size of the particle size distribution, the volume of particles assumed to be spherical is counted and calculated based on the value.

Commercially available starch can be used, such as Tapioca Natural 9096 manufactured by AGRANA, which is tapioca-derived starch; TAPIOCA PURE manufactured by Nouryon Japan Co., Ltd.; A60012 ORGANIC TAPIOCA STARCH POWDER manufactured by Active Concepts; Barley Natural manufactured by AGRANA, which is barley-derived starch; Fine Snow IR manufactured by Joetsu Starch Co., Ltd., which is rice-derived starch; and Resista Natural manufactured by AGRANA.

Examples of the modified starch include starch phosphate and alkylated starch, and examples of the cellulose derivative include cellulose acetate.

Examples of the chitosan include those derived from crabs, shrimps, and krill. Chitosan derived from crabs and shrimps is preferable.

The average particle size of chitosan is preferably 5 to 30 $\mu$m, more preferably 5 to 25 $\mu$m, and even more preferably 5 to 20 $\mu$m. Here, the average particle size of chitosan is specified as in the JIS standard (Z8819-2: 2019).

Chitosan is commercially available; for example, K45, manufactured by Izumi Senko Co., Ltd., is snow crab shell-derived chitosan.

The content of the (A) acyllysine is 3 to 50% by mass, more preferably 5 to 30% by mass, based on 100% by mass of the total amount of the powder composition.

The content of the (B) polymer powder is 5 to 97% by mass, preferably 20 to 95% by mass, and more preferably 30 to 90% by mass, based on 100% by mass of the total amount of the powder composition.

The content of the (C) polymer is 0 to 92% by mass, preferably 5 to 90% by mass, more preferably 10 to 70% by mass, and even more preferably 20 to 60% by mass, based on 100% by mass of the total amount of the powder composition.

In an embodiment in which the powder composition contains cellulose as the (C) polymer, the content of the (A) acyllysine is 3 to 50% by mass, preferably 5 to 25% by mass, the content of the (B) powder is 5 to 97% by mass, preferably 10 to 90% by mass, and the content of the cellulose is 9 to 92% by mass, preferably 30 to 85% by mass, based on 100% by mass of the total amount of the powder composition.

The powder composition of the present invention may further include an inorganic powder.

Examples of the inorganic powder include iron oxide yellow, iron oxide red, iron oxide black, fine particle iron oxide, bismuth oxychloride, zirconium oxide, magnesium oxide, chromium oxide, cobalt oxide, carbon black, ultramarine blue, Prussian blue, zinc oxide, fine particle zinc oxide, titanium oxide, fine particle titanium oxide, silica, porous silica, alumina, cerium oxide, boron nitride, calcium sulfate, barium sulfate, magnesium sulfate, calcium carbonate, magnesium carbonate, aluminum silicate, magnesium silicate, silicon carbide, pigment, lake, sericite, mica, talc, kaolin, clay, bentonite, plate-like barium sulfate, butterfly-like barium sulfate, and hydroxyapatite. These may be used alone or in combination of two or more. The inorganic powder may further be a composite of those described above (for example, silica-coated titanium oxide, micacoated titanium oxide, titanium-coated mica), and those described above may be subjected to surface treatment such as silicone treatment, fluorine compound treatment, silane coupling agent treatment, silane treatment, organic titanate treatment, fatty acid treatment (for example, stearoyl glutamic acid treatment), metal soap treatment (for example, aluminum stearate treatment), oil treatment, and amino acid treatment (for example, silicone-treated talc, silicone-treated mica, silicone-treated sericite, silicone-treated titanium oxide, silicone-treated iron oxide red, silicone-treated iron oxide yellow, silicone-treated iron oxide black, stearoyl glutamate-treated titanium oxide, stearoyl glutamate-treated iron oxide yellow, stearoyl glutamate-treated iron oxide red, stearoyl glutamate-treated iron oxide black, and aluminum stearate-treated titanium oxide). The inorganic powder is preferably silica or calcium carbonate, and more preferably silica.

The average particle size of the inorganic powder is preferably 1 to 30 $\mu$m, more preferably 3 to 25 $\mu$m, and even more preferably 3 to 20 $\mu$m. Here, the average particle size of the inorganic powder is specified as in the JIS standard (Z8819-2: 2019).

The content of the inorganic powder is 0 to 85% by mass, preferably 10 to 84% by mass, more preferably 20 to 83% by mass, and even more preferably 42 to 82% by mass, based on 100% by mass of the total amount of the powder composition.

In an embodiment in which the powder composition of the present invention comprises the (A) acyllysine, the (B) polymer powder, and the inorganic powder, the content of the (A) acyllysine is preferably 3 to 50% by mass, more preferably 8 to 30% by mass, the content of the (B) polymer powder is preferably 6 to 30% by mass, more preferably 7 to 26% by mass, and even more preferably 8 to 23% by mass, and the content of the inorganic powder is 35 to 90% by mass, preferably 37 to 85% by mass, more preferably 40 to 83% by mass, and even more preferably 42 to 82% by mass, based on 100% by mass of the total amount of the powder composition.

The preferred blending of the (A) acyllysine, the (B) polymer powder, the (C) polymer, and the inorganic powder, based on 100% by mass of the total amount of the powder composition, is a blending obtained by optionally combining the preferred blending amounts of each component described in the above paragraphs as the preferred blending amount, depending on the composition of the powder composition, without contradiction.

Specifically, when the powder composition of the present invention comprises (A) acyllysine and the (B) polymer powder and/or the (C) polymer, it is preferable that the content of the (A) is 3 to 50% by mass, the content of the (B) is 5 to 97% by mass, and the content of the (C) is 0 to 92% by mass, it is more preferable that the content of the (A) is 5 to 30% by mass, the content of the (B) is 20 to 95% by mass, and the content of the (C) is 5 to 90% by mass, it is even more preferable that the content of the (A) is 5 to 30% by mass, the content of the (B) is 30 to 90% by mass, and the content of the (C) is 10 to 70% by mass, and it is still more preferable that the content of the (A) is 5 to 30% by mass, the content of the (B) is 30 to 90% by mass, and the content of the (C) is 20 to 60% by mass, based on 100% by mass of the total amount of the powder composition.

When the powder composition of the present invention comprises (A) acyllysine, the (B) polymer powder and/or the (C) polymer, and optionally, the inorganic powder, it is preferable that the content of the (A) is 3 to 50% by mass, the content of the (B) is 0 to 97% by mass, the content of the (C) is 0 to 92% by mass, and the content of the inorganic powder is 0 to 85% by mass, it is more preferable that the content of the (A) is 3 to 50% by mass, the content of the (B) is 5 to 97% by mass, the content of the (C) is 0 to 92% by mass, and the content of the inorganic powder is 0 to 85% by mass, it is even more preferable that the content of the (A) is 5 to 30% by mass, the content of the (B) is 20 to 95% by mass, the content of the (C) is 5 to 90% by mass, and the content of the inorganic powder is 10 to 84% by mass, it is still more preferable that the content of the (A) is 5 to 30% by mass, the content of the (B) is 30 to 90% by mass, the content of the (C) is 10 to 70% by mass, and the content of the inorganic powder is 20 to 83% by mass, and it is yet more preferable that the content of the (A) is 5 to 30% by mass, the content of the (B) is 30 to 90% by mass, the content of the (C) is 20 to 60% by mass, and the content of the inorganic powder is 42 to 82% by mass, based on 100% by mass of the total amount of the powder composition. Alternatively, the content of the (A) may be 3 to 50% by mass, the content of the (B) and/or (C) may be 8 to 23% by mass, and the content of the inorganic powder may be 42 to 89% by mass, based on 100% by mass of the total amount of the powder composition.

When the powder composition of the present invention comprises (A) acyllysine, the (B) polymer powder, and the inorganic powder, it is preferable that the content of the (A) is 3 to 50% by mass, the content of the (B) is 6 to 30% by mass, and the content of the inorganic powder is 35 to 90% by mass, it is more preferable that the content of the (A) is 8 to 30% by mass, the content of the (B) is 7 to 26% by mass, and the content of the inorganic powder is 37 to 85% by mass, it is even more preferable that the content of the (A) is 8 to 30% by mass, the content of the (B) is 8 to 23% by mass, and the content of the inorganic powder is 40 to 83% by mass, and it is still more preferable that the content of the (A) is 5 to 30% by mass, the content of the (B) is 8 to 23% by mass, and the content of the inorganic powder is 42 to 82% by mass, based on 100% by mass of the total amount of the powder composition. Alternatively, the content of the (A) may be 3 to 50% by mass, the content of the (B) may be 8 to 23% by mass, and the content of the inorganic powder may be 42 to 89% by mass, based on 100% by mass of the total amount of the powder composition.

The powder composition of the present invention preferably has a structure in which the (B) polymer powder and/or the (C) polymer is a core (nucleus) particle and the (A) acyllysine is attached to the surface thereof. When the powder composition of the present invention further comprises an inorganic powder, it preferably has a structure in which the (B) polymer powder and/or the (C) polymer and the inorganic powder are core (nucleus) particles and the (A) acyllysine is attached to the surface thereof. When the (A) acyllysine is attached to the surface of the core (nucleus) particles, the powder composition of the present invention can exhibit excellent properties such as a soft feel, sliminess, and further a moist feel, adhesion to the skin, and a silky feel. In order for the (A) acyllysine to adhere to the surface of the core (nucleus) particles, the median diameter of volume-based distribution of the (A) acyllysine is 9 $\mu$m or less, so that the adhesion of the acyllysine to the core (nucleus) particles is enhanced. When the (A) acyllysine is attached to the surface of the core (nucleus) particles, the water repellency of the powder composition is enhanced.

The powder composition of the present invention comprises (A) acyllysine, the (B) polymer powder and/or the (C) polymer, and optionally, the inorganic powder, and has a water-repellent time using water of preferably 30 minutes or more, more preferably 60 minutes or more.

The water-repellent time using water is the time it takes for the residual ratio of the powder floating on water to be less than 90% after 5 g of water is placed in a 10 mL glass vial and 30 mg of the powder composition is placed on the liquid surface.

The median diameter of volume-based distribution of the powder composition of the present invention is preferably 5 to 39 $\mu$m, more preferably 5 to 30 $\mu$m, and even more preferably 5 to 20 $\mu$m. The area circularity of the powder composition of the present invention is preferably 0.62 or more, and more preferably 0.68 or more.

The powder composition of the present invention can be obtained by mixing the (A) acyllysine and the (B) polymer powder. The (A) acyllysine and the (B) polymer powder can also be charged and mixed simultaneously. When mixing the (A) acyllysine and the (B) polymer powder, the inorganic powder can also be mixed, and the inorganic powder can also be charged with the (A) acyllysine and the (B) polymer powder and mixed simultaneously.

When the powder composition of the present invention comprises the (C) polymer, the powder composition of the present invention can be obtained by mixing the (A) acyllysine, the (B) polymer powder, and the (C) polymer. The (A) acyllysine, the (B) polymer powder, and the (C) polymer can also be charged and mixed simultaneously. When mixing the (A) acyllysine, the (B) polymer powder, and the (C) polymer, the inorganic powder can also be mixed, and the inorganic powder can also be charged with the (A) acyllysine, the (B) polymer powder, and the (C) polymer and mixed simultaneously.

The above mixing can be carried out by dry mixing. The dry mixing in the present invention includes not only an embodiment in which no solvent is used at all but also an embodiment in which a small amount of solvent is used.

The mixing can be carried out by mixing with a mixer. The mixing time is preferably 120 minutes or less from the viewpoint that the production can be performed at low cost without reducing productivity. The mixing time is preferably 1 minute or more, and more preferably 10 minutes or more. Usable mixers include high-speed stirring type mixers such as Henschel mixers, household mixers, or high-shear mixers; container rotation type mixers or container rotation type mixers with stirrers such as W-type mixers, CV-type mixers, V-type mixers, or rocking mixers; mechanical stirring type mixers such as ribbon stirring types, multi-shaft paddle types, twin-screw types, or conical screw types; and compression, shearing, and impact type mixers such as airflow stirring type mixers, Julia mixers, Nauta mixers, or Nobilta mixers; and high-speed stirring type mixers are preferable from the viewpoints of inexpensive production and general versatility.

A powder composition comprising the (C) polymer having a sugar or glucosamine as a monomer and the (A) acyllysine having a median diameter of volume-based distribution of 9 μm or less is also a powder composition capable of providing a soft feel and sliminess. In the powder composition comprising the (C) polymer and the (A) acyllysine, the content of the (A) acyllysine is 3 to 50% by mass, more preferably 5 to 30% by mass, based on 100% by mass of the total amount of the powder composition. The content of the (C) polymer is 1 to 92% by mass, more preferably 5 to 85% by mass, and even more preferably 8 to 80% by mass, based on 100% by mass of the total amount of the powder composition. In the powder composition comprising the (C) polymer and the (A) acyllysine, the (C) polymer is preferably spherical or substantially circular among the above-mentioned polymers. The spherical or substantially circular starch is preferably starch derived from rice, tapioca, or barley. The spherical or substantially circular cellulose is preferably cellulose having a particle size of 5 μm to 12 μm.

The powder composition comprising the (C) polymer and the (A) acyllysine may further include the inorganic powder.

The powder composition comprising the (C) polymer and the (A) acyllysine can be obtained by mixing the (A) acyllysine and the (C) polymer. The (A) acyllysine and the (C) polymer can also be charged and mixed simultaneously. When mixing the (A) acyllysine and the (C) polymer, the inorganic powder can also be mixed, and the inorganic powder can also be charged with the (A) acyllysine and the (C) polymer and mixed simultaneously.

The mixing can be carried out by dry mixing. The dry mixing includes not only an embodiment in which no solvent is used at all but also an embodiment in which a small amount of solvent is used.

The mixing can be carried out by mixing with a mixer. The mixing time is preferably 120 minutes or less from the viewpoint that the production can be performed at low cost without reducing productivity. The mixing time is preferably 1 minute or more, and more preferably 10 minutes or more. Usable mixers include the mixers listed above.

The powder composition comprising the (C) polymer and the (A) acyllysine can also be used as cosmetics or external preparations for skin. The cosmetics or external preparations for skin are as described above.

The composition of the present invention can also be used as cosmetics or external preparations for skin. The cosmetics or external preparations for skin can be prepared into any form of preparation applicable to a desired part (e.g., skin, hair, scalp, lips, eyes, eyelashes, eyelids, nails) according to a conventional method. Examples of the cosmetics or external preparations for skin, lips, eyelashes, and nails include sunscreens such as sunscreen, body powders, and sprays; makeup cosmetics such as foundations, face powders, primers, BB creams, body colors, bronzers, face powders, loose powders, nail polishes, cheek colors, makeup bases, and concealers; lip cosmetics such as lip colors, lip liners, and lipsticks; eye makeup cosmetics such as eyeliners, eye shadows, eyebrows products, and mascaras; leave-on cosmetics such as milky lotions, lotions, creams, gels, and serums; and face masks. Examples of the cosmetics or external preparations for hair include hair styling agents, hair milks, hair treatments, hair conditioners, and hair lotions. Examples of the cosmetics or external preparations for scalp include hair growth agents. Examples of preferable cosmetics include makeup cosmetics, eye makeup cosmetics, lip cosmetics, and leave-on cosmetics. Examples of preferable external preparations include ointments, creams, mousses, and gels. Particularly preferred applications of the present invention include makeup cosmetics.

When used as cosmetics or external preparations for skin, the cosmetics may usually contain components that can be used in cosmetics (including topical pharmaceutical preparations and quasi-drugs), as long as the effects of the present invention are not impaired. Examples thereof include oil agents, inorganic powders, water, surfactants, amino acids, amino acid derivatives, lower alcohols (for example, ethanol), polyhydric alcohols (for example, glycerin, butylene glycol), sugar alcohols and alkylene oxide adducts thereof, water-soluble polymers (for example, hydroxyethyl cellulose), film-forming polymers, gelling agents (for example, dibutyl lauroyl glutamide, dibutyl ethylhexanoyl glutamide), humectants (for example, sodium pyrrolidone carboxylate), bactericides and antibacterial agents, anti-inflammatory agents, analgesics, antifungal agents, keratolytic exfoliating agents, skin coloring agents, hormonal agents, ultraviolet absorbers, hair growth agents, antiperspirants and astringent active ingredients (for example, zinc pyrrolidone carboxylate), antiperspirant deodorants, vitamin agents, blood flow promoting agents (vasodilators, blood circulation promoters), herbal medicines, plant extracts, pH adjusters, chelating agents (for example, EDTA-2Na), viscosity modifiers, pearlizing agents, natural fragrances, synthetic fragrances, dyes and pigments (for example, Red No. 202 and Blue No. 1), antioxidants (for example, tocopherol, tocopherol acetate, and pentagalloyl glucoside), preservatives (for example, methylparaben, butylparaben, propylparaben, and phenoxyethanol), emulsifiers, thickeners, fats and waxes, silicone compounds, and fragrant oils.

Examples of oil agents include higher alcohols such as octyldodecanol and oleyl alcohol; hydrocarbon oils such as squalane, liquid paraffin, hydrogenated polyisobutene, and isododecane; natural or synthetic ester oils such as jojoba seed oil, isononyl isononanoate, isostearyl neopentanoate, cetyl 2-ethylhexanoate, ethylhexyl palmitate, alkyl benzoate, polyglyceryl-2 tetraisostearate, phytosteryl/octyldodecyl lauroyl glutamate, isopropyl lauroyl sarcosinate, ethylhexyl methoxycinnamate, phytosteryl/decyl tetradecyl myristoyl methyl beta-alanine, glyceryl caprate; diglycerides; natural or synthetic triglycerides such as corn oil, olive oil, sunflower oil, caprylic/capric triglyceride, triethylhexanoin; higher viscosity oils such as phytosteryl/isostearyl/cetyl/stearyl/behenyl dimer dilinoleate, diisostearyl malate, hydrogenated polydecene, polyglyceryl-2 triisostearate, polyglyceryl-2 diisostearate, pentaerythrityl tetraisostearate; silicone oils such as dimethicone, methicone, cyclopentasiloxane, cyclohexasiloxane, phenyl trimethicone, PEG-10 dimethicone; fluorinated oils such as perfluoropolyether, perfluorodecalin, perfluorooctane; mineral oil; etc. These may be used alone or in combination of two or more.

Examples of the inorganic powder include iron oxide yellow, iron oxide red, iron oxide black, fine particle iron oxide, bismuth oxychloride, zirconium oxide, magnesium oxide, chromium oxide, cobalt oxide, carbon black, ultramarine blue, Prussian blue, zinc oxide, fine particle zinc oxide, titanium oxide, fine particle titanium oxide, silica, porous silica, alumina, cerium oxide, boron nitride, calcium sulfate, barium sulfate, magnesium sulfate, calcium carbonate, magnesium carbonate, aluminum silicate, magnesium silicate, silicon carbide, pigment, lake, sericite, mica, talc, kaolin, clay, bentonite, plate-like barium sulfate, butterfly-like barium sulfate, and hydroxyapatite. These may be used alone or in combination of two or more. The inorganic powder may further be a composite of those described above (for example, silica-coated titanium oxide, micacoated titanium oxide, titanium-coated mica), and those described above may be subjected to surface treatment such as silicone treatment, fluorine compound treatment, silane coupling agent treatment, silane treatment, organic titanate treatment, fatty acid treatment (for example, stearoyl glutamic acid treatment), metal soap treatment (for example, aluminum stearate treatment), oil treatment, and amino acid treatment (for example, silicone-treated talc, silicone-treated mica, silicone-treated sericite, silicone-treated titanium oxide, silicone-treated iron oxide red, silicone-treated iron oxide yellow, silicone-treated iron oxide black, stearoyl glutamate-treated titanium oxide, stearoyl glutamate-treated iron oxide yellow, stearoyl glutamate-treated iron oxide red, stearoyl glutamate-treated iron oxide black, and aluminum stearate-treated titanium oxide).

Note that when the composition of the present invention is used as a cosmetic or as an external preparation for skin, the inorganic powder referred to in this paragraph is intended to be blended with the composition, with the usual components that can be used in cosmetics (including topical pharmaceutical preparations and quasi-drugs), as long as the effects of the present invention are not impaired; the inorganic powder may be the same as or different from the inorganic powder optionally blended with the composition of the present invention described in paragraph [0019].

Examples

(Examples 1 to 17, Comparative Examples 1 to 6, and Reference Examples 3, 4, and 6 to 9)

Each of the raw materials was weighed in the blending ratios (parts by mass) shown in Tables 1 to 4 so that the total amount was 5.0 g, and all the raw materials were simultaneously charged into a lab mill (Tescom Mill & Mixer TML162). The mixture was mixed for 100 seconds (20 seconds five times, with the powder adhering to the inner wall surface scraped off between each mixing) to obtain an evaluation sample.

Sensory evaluation method:

An interview survey was conducted with a panel of five experts on the evaluation items of softness and sliminess, and when the score of the comparative control (Comparative Example 6) was 5, each evaluation item was scored by the following method, and the average score of the five panelists was used to evaluate each evaluation item in 5 grades from A to E. For sliminess, a smooth and meltable feel on the skin was evaluated. For silky feel, a uniform spreadability during application and a high feeling of adhesion to the skin were evaluated. The evaluation results are shown in Tables 1 to 4.

(Score): (Evaluation)

6: Very good
5: Good
4: Slightly good

3: Slightly poor
2: Poor
1: Very poor

(Judgment): (Average score)

A: 5.0 or more
B: 4.4 to 4.9
C: 3.9 to 4.3
D: 3.1 to 3.8
E: 3.0 or less

Biodegradability evaluation method:

Those satisfying OCED 306, ISO 17088, ISO-TG301F, or ASTM D6400 were marked with O, and those not satisfying were marked with ×. The evaluation results are shown in Tables 1 to 4.

Table 1

| | Component Name | Example 1 | Example 2 | Example 3 | Example 4 | Example 5 | Example 6 | Example 7 | Example 8 | Example 9 |
|---|---|---|---|---|---|---|---|---|---|---|
| Component A | Lauroyl Lysine 1 | 15 | 15 | 15 | 15 | 15 | 15 | 15 | 15 | 15 |
| Component B | Silk Powder | 100 | | | | 75 | 75 | 75 | | |
| | Polylactic Acid | | 100 | | | | | | 75 | |
| | PHB | | | 100 | | | | | | 75 |
| | PHBV | | | | 100 | | | | | |
| Component C | Tapioca-Derived Starch | | | | | 25 | | | 25 | 25 |
| | Barley-Derived Starch | | | | | | 25 | | | |
| | Rice-Derived Starch | | | | | | | 25 | | |
| Eval. Item | Soft Feel | A | B | A | A | A | A | A | A | A |
| | Sliminess | A | A | A | A | A | A | A | A | A |
| | Silky Feel | A | A | A | A | A | A | A | A | A |
| | Biodegradability | O | O | O | O | O | O | O | O | O |

EP 4 501 307 A1

Table 2

| | Component Name | Example 10 | Example 11 | Example 12 | Example 13 | Example 14 | Example 15 | Example 16 | Example 17 |
|---|---|---|---|---|---|---|---|---|---|
| Component A | Lauroyl Lysine 1 | 15 | 15 | 15 | 15 | 15 | 5 | 15 | 15 |
| Component B | Silk Powder | | 50 | 10 | | | 100 | | 70 |
| | Polylactic Acid | | | | 50 | 10 | | | |
| | PHBV | 75 | | | | | | | |
| | Calcium Alginate | | | | | | | 100 | |
| Component C | Crystalline Cellulose 2 | | | | | | | | 30 |
| | Tapioca-Derived Starch | 25 | 50 | 90 | 50 | 90 | | | |
| Eval. Item | Soft Feel | A | A | A | A | A | A | A | A |
| | Sliminess | A | B | A | B | B | B | A | A |
| | Silky Feel | A | A | A | A | A | A | A | A |
| | Biodegradability | ○ | ○ | ○ | ○ | ○ | ○ | ○ | ○ |

12

EP 4 501 307 A1

Table 3

| | Component Name | Comparative Example 1 | Comparative Example 2 | Comparative Example 3 | Comparative Example 4 | Comparative Example 5 | Comparative Example 6 |
|---|---|---|---|---|---|---|---|
| Component A | Lauroyl Lysine 2 | 15 | 15 | | | 15 | |
| Component B | Silk Powder | | | 100 | | | |
| | Polylactic Acid | | | | 100 | | |
| Component C | Crystalline Cellulose 2 | | | | | 100 | |
| | Crystalline Cellulose 3 | | 100 | | | | |
| | Com-Derived Starch | 100 | | | | | |
| | Nylon Fine Particles | | | | | | 100 |
| Eval. Item | Soft Feel | D | D | C | D | D | A |
| | Sliminess | E | E | D | E | D | A |
| | Silky Feel | D | D | D | D | D | B |
| | Biodegradability | ○ | ○ | ○ | ○ | ○ | × |

Table 4

| | Component Name | Reference Example 3 | Reference Example 4 | Reference Example 6 | Reference Example 7 | Reference Example 8 | Reference Example 9 |
|---|---|---|---|---|---|---|---|
| Component A | Lauroyl Lysine 1 | 15 | 15 | 15 | 15 | 15 | 15 |
| | Crystalline Cellulose 1 | | | | | 100 | |
| | Crystalline Cellulose 2 | | | 100 | | | |
| | Crystalline Cellulose 3 | | | | 100 | | |
| Component C | Tapioca-Derived Starch | | | | | | |
| | Barley-Derived Starch | | | | | | |
| | Rice-Derived Starch | 100 | | | | | |
| | Corn-Derived Starch | | 100 | | | | |
| | Potato-Derived Starch | | | | | | |
| | Chitosan | | | | | | 100 |
| Eval. Item | Soft Feel | B | C | B | B | B | A |
| | Sliminess | B | C | B | B | B | B |
| | Silky Feel | A | C | A | A | C | B |
| | Biodegradability | O | O | O | O | O | O |

(Examples A1 to A16, Comparative Examples A1 to A25, and Reference Examples A1 to A4)

Each of the raw materials was weighed in the blending ratios (parts by mass) shown in Tables 5 to 11 so that the total amount was 5.0 g, and all the raw materials were simultaneously charged into a lab mill (Tescom Mill & Mixer TML162). The mixture was mixed for 100 seconds (20 seconds five times, with the powder adhering to the inner wall surface scraped off between each mixing) to obtain an evaluation sample.

Sensory evaluation method:

An interview survey was conducted with a panel of five experts on the evaluation items of softness and sliminess, and when the score of the comparative control (Comparative Example 6) was 5, each evaluation item was scored by the following method, and the average score of the five panelists was used to evaluate each evaluation item in 5 grades from A to E. For sliminess, a smooth and meltable feel on the skin was evaluated. For silky feel, a uniform spreadability during application and a high feeling of adhesion to the skin were evaluated. For rolling feel, it was evaluated that the powder moves around on the skin as particles, slides well, and feels less frictional. For moist feel, it was evaluated that it feels as if it contains an appropriate amount of water. The evaluation results are shown in Tables 5 to 10.

(Score): (Evaluation)

6: Very good
5: Good
4: Slightly good
3: Slightly poor
2: Poor
1: Very poor

(Judgment): (Average score)

A: 5.0 or more
B: 4.4 to 4.9
C: 3.9 to 4.3
D: 3.1 to 3.8
E: 3.0 or less

Biodegradability evaluation method:

Those satisfying OCED 306, ISO 17088, ISO-TG301F, or ASTM D6400 were marked with O, and those not satisfying were marked with ×. The evaluation results are shown in Tables 1 to 4.

Measuring method of median diameter of starch:

The particle size distribution was measured using a laser diffraction/scattering particle size distribution analyzer (Partica LA-950, manufactured by HORIBA, Ltd.) using the volume cumulative value. The particle size of the volume-based distribution was determined by analyzing the measurement results using software attached to the apparatus.
Starch in an amount of 20 mg to be measured was added to 5 g of ultrapure water (Milli-Q water), and the mixture was dispersed by ultrasonicating for 30 minutes while stirring with an ultrasonic device having an output of 300 Watt. An appropriate amount of the dispersion was added to 500 mL of ultrapure water (Milli-Q water) according to the procedure of the apparatus, and a dispersion sample having an appropriate concentration was prepared while checking the transparency. The sample was dispersed into primary particles by applying ultrasonic waves for 30 minutes while circulating at a flow rate of 10 mL/min, degassed, and then the particle size distribution and particle size of the starch in the sample were determined using a flow cell.

Measurement of area circularity:

The area circularity was evaluated using a fully automatic image analysis particle size distribution analyzer (Morphologi G3, manufactured by Malvern Instruments Ltd.). Specifically, 7 mm$^3$ of a measurement sample was weighed (scraped off with a spatula), and the powder was dispersed under the powder dispersion conditions (dispersion pressure: 5.0 bar, dispersion time: 20 ms). The measurement was performed using a 10x objective lens. The circularity of each of 20000 particles was subjected to image analysis, and the average value was adopted as the circularity of each sample.

Water repellency evaluation method:

Water in an amount of 5 g was measured into a vial, and 30 mg of various treated powders were added to the water surface from a height of 3 cm within 2 seconds. After standing for 60 minutes after the addition, the residual ratio of the powder floating on the solvent was visually observed and analyzed using ImageJ, and the case where the residual ratio was 90% or more was marked with O, and the case where it was less than 90% was marked with ×.

Table 5

| | Component Name | Comparative Example A1 | Comparative Example A2 | Comparative Example A3 | Comparative Example 4A | Comparative Example A5 | Comparative Example A6 |
|---|---|---|---|---|---|---|---|
| Component A | Lauroyl Lysine 1 | | | | | | |
| | Crystalline Cellulose 1 | 90 | 90 | 53 | 53 | 10 | 10 |
| | Cellulose 1 | | | | | | |
| | Tapioca-Derived Starch | 10 | | 47 | | 90 | |
| Component C | Barley-Derived Starch | | 10 | | 47 | | 90 |
| | Potato-Derived Starch | | | | | | |
| | Rice-Derived Starch | | | | | | |
| | Com-Derived Starch | | | | | | |
| Eval. Item | Soft Feel | B | B | B | B | B | B |
| | Sliminess | B | B | B | B | B | B |
| | Silky Feel | C | C | C | C | C | C |
| | Rolling Feel | C | C | C | C | C | C |
| | Moist Feel | C | C | B | B | B | B |
| | Biodegradability | ○ | ○ | ○ | ○ | ○ | ○ |
| | Starch Particle Size [p,m] | 15 | 16 | 15 | 16 | 15 | 16 |
| | Area Circularity | | | | | | |
| | Water Repellency Eval. | × | × | × | × | × | × |

Table 6

| | Component Name | Comparative Example A7 | Comparative Example A8 | Comparative Example A9 | Comparative Example A10 | Comparative Example A11 | Comparative Example A12 |
|---|---|---|---|---|---|---|---|
| Component A | Lauroyl Lysine 1 | | | | | | |
| Component C | Crystalline Cellulose 1 | | | | | | |
| | Cellulose 1 | 90 | 90 | 53 | 53 | 10 | 10 |
| | Tapioca-Derived Starch | 10 | | 47 | | 90 | |
| | Barley-Derived Starch | | 10 | | 47 | | 90 |
| | Potato-Derived Starch | | | | | | |
| | Rice-Derived Starch | | | | | | |
| | Com-Derived Starch | | | | | | |
| Eval. Item | Soft Feel | A | A | A | A | A | A |
| | Sliminess | A | B | A | A | A | A |
| | Silky Feel | C | C | C | C | C | C |
| | Rolling Feel | A | B | B | B | A | B |
| | Moist Feel | B | B | B | B | A | A |
| | Biodegradability | ○ | ○ | ○ | ○ | ○ | ○ |
| | Starch Particle Size [μm] | 15 | 16 | 15 | 16 | 15 | 16 |
| | Area Circularity | | | | | | |
| | Water Repellency Eval. | × | × | × | × | × | × |

EP 4 501 307 A1

Table 7

| | Component Name | Reference Example A1 | Reference Example A2 | Example A1 | Example A2 | Comparative Example A13 |
|---|---|---|---|---|---|---|
| Component A | Lauroyl Lysine 1 | 15 | 15 | 15 | 15 | 15 |
| Component C | Crystalline Cellulose 1 | 100 | | | | |
| | Cellulose 1 | | 100 | | | |
| | Tapioca-Derived Starch | | | 100 | | |
| | Barley-Derived Starch | | | | 100 | |
| | Potato-Derived Starch | | | | | 100 |
| | Rice-Derived Starch | | | | | |
| | Com-Derived Starch | | | | | |
| Eval. Item | Soft Feel | B | B | B | B | C |
| | Sliminess | B | B | B | A | C |
| | Silky Feel | B | B | A | B | C |
| | Rolling Feel | C | A | B | B | C |
| | Moist Feel | B | B | B | B | C |
| | Biodegradability | O | O | O | O | O |
| | Starch Particle Size [μm] | - | - | 15 | 16 | 40 |
| | Area Circularity | | | 0.642 | | |
| | Water Repellency Eval. | O | O | O | O | O |

Table 8

| | Component Name | Comparative ExampleA14 | Comparative ExampleA15 | Comparative Example A16 | Example A3 | Example A4 | Comparative Example A17 |
|---|---|---|---|---|---|---|---|
| Component A | Lauroyl Lysine 1 | 15 | 15 | 15 | 15 | 15 | 15 |
| Component C | Crystalline Cellulose 1 | 90 | 90 | 90 | 53 | 53 | 53 |
| | Cellulose 1 | | | | | | |
| | Tapioca-Derived Starch | 10 | | | 47 | | |
| | Barley-Derived Starch | | 10 | | | 47 | |
| | Potato-Derived Starch | | | 10 | | | 47 |
| | Rice-Derived Starch | | | | | | |
| | Com-Derived Starch | | | | | | |
| Eval. Item | Soft Feel | B | B | B | B | B | B |
| | Sliminess | B | B | C | B | B | C |
| | Silky Feel | B | B | C | B | B | C |
| | Rolling Feel | C | C | C | B | B | C |
| | Moist Feel | C | C | C | B | B | C |
| | Biodegradability | ○ | ○ | ○ | ○ | ○ | ○ |
| | Starch Particle Size [pm] | 15 | 16 | 40 | 15 | 16 | 40 |
| | Area Circularity | 0.596 | 0.543 | | 0.663 | 0.611 | |
| | Water Repellency Eval. | ○ | ○ | ○ | ○ | ○ | ○ |

Table 9

| | Component Name | Example A5 | Example A6 | Comparative Example A18 | Example A7 | Example A8 | Comparative Example A19 |
|---|---|---|---|---|---|---|---|
| Component A | Lauroyl Lysine 1 | 15 | 15 | 15 | 15 | 15 | 15 |
| Component C | Crystalline Cellulose 1 | 10 | 10 | 10 | | | |
| | Cellulose 1 | | | | 90 | 90 | 90 |
| | Tapioca-Derived Starch | 90 | | | 10 | | |
| | Barley-Derived Starch | | 90 | | | 10 | |
| | Potato-Derived Starch | | | 90 | | | 10 |
| | Rice-Derived Starch | | | | | | |
| | Com-Derived Starch | | | | | | |
| Eval. Item | Soft Feel | B | B | B | A | A | A |
| | Sliminess | B | B | C | A | B | B |
| | Silky Feel | B | B | C | A | B | C |
| | Rolling Feel | A | B | C | A | B | C |
| | Moist Feel | B | B | C | B | B | B |
| | Biodegradability | O | O | O | O | O | O |
| | StarchParticle Size [$\mu$m] | 15 | 16 | 40 | 15 | 16 | 40 |
| | Area Circularity | 0.686 | 0.628 | | 0.711 | 0.641 | |
| | Water Repellency Eval. | O | O | O | O | O | O |

Table 10

| | Component Name | Example A9 | Example A1O | Comparative Example A20 | Reference Example A3 | Reference Example A4 | Example A11 | Example A12 | Comparative Example A21 |
|---|---|---|---|---|---|---|---|---|---|
| Component A | Lauroyl Lysine 1 | 15 | 15 | 15 | 15 | 15 | 15 | 15 | 15 |
| Component C | Crystalline Cellulose 1 | | | | | | 10 | 10 | 10 |
| | Cellulose 1 | 53 | 53 | 53 | 53 | 53 | | | |
| | Tapioca-Derived Starch | 47 | | | | | 90 | | |
| | Barley-Derived Starch | | 47 | | | | | 90 | |
| | Potato-Derived Starch | | | 47 | | | | | 90 |
| | Rice-Derived Starch | | | | 47 | | | | |
| | Com-Derived Starch | | | | | 47 | | | |
| Eval. Item | Soft Feel | A | A | A | A | A | A | A | B |
| | Sliminess | A | A | A | B | A | A | A | B |
| | Silky Feel | A | B | C | B | B | A | A | C |
| | Rolling Feel | B | B | C | B | B | A | B | C |
| | Moist Feel | B | B | C | A | B | A | A | C |
| | Biodegradability | O | O | O | O | O | O | O | O |
| | Starch Particle Size [μm] | 15 | 16 | 40 | 8 | 16 | 15 | 16 | 40 |
| | Area Circularity | 0.644 | | | | | 0.690 | 0.620 | |
| | Water Repellency Eval. | O | O | O | O | O | O | O | O |

Table 11

| | Component Name | Comparative ExampleA22 | Example A13 | Examp1eA14 | Comparative Example A23 | Comparative ExampleA24 | ExampleA15 | Example A16 | Comparative Example A25 |
|---|---|---|---|---|---|---|---|---|---|
| Component A | Lauroyl Lysine 1 | 10 | 10 | 10 | 10 | 1 | 3 | 50 | 60 |
| Inorganic Powder | Silica 1 | 86 | 82 | 67 | 60 | 91 | 89 | 42 | 32 |
| Component B | Silk Powder | 4 | 8 | 23 | 30 | 8 | 8 | 8 | 8 |
| Eval. Item | Soft Feel | B | A | A | A | C | B | A | A |
| | Sliminess | C | A | A | A | B | B | A | A |
| | Silky Feel | C | B | A | A | C | B | A | A |
| | Moist Feel | C | B | A | A | B | B | A | A |
| | Rolling Feel | A | A | B | C | A | A | B | C |
| | Biodegradability | | | | | | | | |
| | Water Repellency Eval. | ○ | ○ | ○ | ○ | × | ○ | ○ | ○ |

EP 4 501 307 A1

Raw materials:

The raw materials used in the Examples, Comparative Examples, and Reference Examples in Tables 1 to 4 are as follows.

Lauroyl lysine 1 (component A) (median diameter of volume-based distribution: 3.9 $\mu$m, bulk density: 0.36 g/mL)

Lauroyl lysine 2: AMIHOPE (trademark) LL (median diameter of volume-based distribution: 14.3 $\mu$m, bulk density: 0.42 g/mL) sold by Ajinomoto Co., Inc.

Silk powder (component B): Silk Powder S15 (average particle size: 8 $\mu$m) manufactured by Izumi Senko Co., Ltd.

Polylactic acid (component B): Ecosoft 608XF (average particle size: 10 $\mu$m) manufactured by Micro Powders, Inc.

Polyhydroxybutyric acid (PHB) (component B): Biosoft 915 (average particle size: 10 $\mu$m) manufactured by Micro Powders, Inc.

Poly(3-hydroxybutyric acid-co-3-hydroxyvaleric acid) (PHBV) (component B): NatureMatte 31 (average particle size: 9 $\mu$m) manufactured by Micro Powders, Inc.

Polymer powder of calcium alginate (component B): Flavikafine (average particle size: 5 $\mu$m) manufactured by Nisshinbo Holdings Inc.

Crystalline cellulose 1 (component C): VIVAPUR CS 4 (average particle size: 4 $\mu$m) manufactured by J. Rettenmaier & Söhne (JRS)

Crystalline cellulose 2 (component C): VIVAPUR CS 5 (average particle size: 5 $\mu$m) manufactured by J. Rettenmaier & Söhne (JRS)

Crystalline cellulose 3 (component C): VIVAPUR CS 12 (average particle size: 12 $\mu$m) manufactured by J. Rettenmaier & Söhne (JRS)

Cellulose 1 (component C): CELLULOBEADS D5 (average particle size: 5 $\mu$m) manufactured by Daito Kasei Kogyo Co., Ltd.

Tapioca-derived starch (component C): Tapioca Natural 9096 (average particle size: 15 $\mu$m) manufactured by AGRANA

Barley-derived starch (component C): Barley Natural (average particle size: 16 $\mu$m) manufactured by AGRANA

Rice-derived starch (component C): Fine Snow IR (average particle size: 8 $\mu$m) manufactured by Joetsu Starch Co., Ltd.

Corn-derived starch: ST Starch C (average particle size: 16 $\mu$m) manufactured by Nippon Starch Chemical Co., Ltd.

Potato-derived starch (component C): ST Starch P (average particle size: 40 $\mu$m) manufactured by Nippon Starch Chemical Co., Ltd.

Nylon fine particles: Nylon 12 true spherical fine particle grade >SP-500 (average particle size: 5 $\mu$m) manufactured by Toray Industries, Inc.

Chitosan: K45 manufactured by Izumi Senko Co., Ltd.

Silica 1: Silica microbead P-1500 (average particle size: 9 $\mu$m) manufactured by JGC Catalysts and Chemicals Ltd.

**Claims**

1. A powder composition comprising

   (A) acyllysine having a median diameter of volume-based distribution of 9 $\mu$m or less; and
   (B) a powder of a polymer having at least one selected from amino acids, hydroxycarboxylic acids, uronic acids, polyvalent carboxylic acids, and polyhydric alcohols as a monomer; and/or (C) a polymer having a sugar or glucosamine as a monomer.

2. The composition according to claim 1, wherein the polymer of (B) is at least one selected from proteins, hydroxycarboxylic acid-based polyesters, polyhydroxyalkanoic acids, diol carboxylic acid-based polyesters, and alginic acid.

3. The composition according to claim 1 or 2, wherein the (A) acyllysine is lauroyl lysine or octanoyl lysine.

4. The composition according to any one of claims 1 to 3, comprising (C) a polymer having a sugar or glucosamine as a monomer.

5. The composition according to any one of claims 1 to 4, wherein the (C) is at least one selected from cellulose, starch, chitin, and chitosan.

6. The composition according to any one of claims 1 to 5, wherein the polymer of the (B) powder exhibits biodegradability in any one of ASTM D6400 biodegradability test, ISO 17088 test method, ISO-TG301F test method, and OCED 306 test method.

7. The composition according to any one of claims 1 to 6, wherein the (A) acyllysine has a bulk density of 0.38 g/mL or less.

8. The composition according to any one of claims 1 to 7, wherein

> the content of the (A) is 3 to 50% by mass,
> the content of the (B) is 5 to 97% by mass, and
> the content of the (C) is 0 to 92% by mass,
> based on 100% by mass of the total amount of the powder composition.

9. The composition according to any one of claims 1 to 8, further comprising an inorganic powder.

10. The composition according to claim 9, wherein

> the content of the (A) is 3 to 50% by mass,
> the content of the (B) is 0 to 97% by mass,
> the content of the (C) is 0 to 92% by mass, and
> the content of the inorganic powder is 0 to 85% by mass,
> based on 100% by mass of the total amount of the powder composition.

11. The composition according to claim 9 or 10, wherein

> the content of the (A) is 3 to 50% by mass,
> the content of the (B) is 6 to 30% by mass, and
> the content of the inorganic powder is 35 to 90% by mass,
> based on 100% by mass of the total amount of the powder composition.

12. A method for producing the composition according to any one of claims 1 to 11, comprising mixing:

> (A) acyllysine having a median diameter of volume-based distribution of 9 $\mu$m or less,
> (B) a powder of a polymer having at least one selected from amino acids, hydroxycarboxylic acids, uronic acids, polyvalent carboxylic acids, and polyhydric alcohols as a monomer, and/or (C) a polymer having a sugar or glucosamine as a monomer, and

> optionally, an inorganic powder.

13. A method for producing the composition according to any one of claims 1 to 11, comprising mixing (A) acyllysine having a median diameter of volume-based distribution of 9 $\mu$m or less, (B) a powder of a polymer having at least one selected from amino acids, hydroxycarboxylic acids, uronic acids, polyvalent carboxylic acids, and polyhydric alcohols as a monomer, and (C) a polymer having a sugar or glucosamine as a monomer.

14. The production method according to claim 12 or 13, wherein the mixing comprises mixing by dry mixing.

15. The production method according to any one of claims 12 to 14, wherein the mixing comprises mixing with a mixer for 120 minutes or less.

## INTERNATIONAL SEARCH REPORT

| International application No. |
|---|
| **PCT/JP2023/013708** |

**A. CLASSIFICATION OF SUBJECT MATTER**

*A61K 8/44*(2006.01)i; *A61K 8/72*(2006.01)i; *A61Q 1/00*(2006.01)i; *A61Q 19/00*(2006.01)i
FI: A61K8/44; A61K8/72; A61Q19/00; A61Q1/00

According to International Patent Classification (IPC) or to both national classification and IPC

**B. FIELDS SEARCHED**

Minimum documentation searched (classification system followed by classification symbols)

A61K8/44; A61K8/72; A61Q1/00; A61Q19/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2010-163369 A (KAO CORP.) 29 July 2010 (2010-07-29) paragraphs [0001], [0005], [0042]-[0045], [0059], [0061] | 1-15 |
| Y | WO 2020/262367 A1 (AJINOMOTO CO., INC.) 30 December 2020 (2020-12-30) claims, paragraph [0009] | 1-15 |
| Y | JP 2000-63229 A (KOSE CORP.) 29 February 2000 (2000-02-29) paragraphs [0004], [0023], [0024], [0034], [0040] | 1-15 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 June 2023** | **13 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2023/013708**

| Patent document cited in search report | | | Publication date (day/month/year) | Patent family member(s) | Publication date (day/month/year) |
|---|---|---|---|---|---|
| JP | 2010-163369 | A | 29 July 2010 | (Family: none) | |
| WO | 2020/262367 | A1 | 30 December 2020 | US 2022/0119343 A1 claims, paragraph [0014] EP 3992175 A1 | |
| JP | 2000-63229 | A | 29 February 2000 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5662937 A
- JP 2019030775 A
- JP 2020075878 A
- WO 2020262367 A